# EUROPÄISCHE PATENTSCHRIFT

(11) **EP 0 742 199 B1**
(45) Veröffentlichungstag und Bekanntmachung des Hinweises auf die Patenterteilung: **11.08.1999**
(21) Anmeldenummer: 96106875.6
(22) Anmeldetag: 02.05.1996
(51) Int. Cl.: C07C 209/16, C07C 211/07, C07C 211/08

(54) **Herstellung von primären Guerbetaminen**
Preparation of primary guerbet amines
Préparation d'amines primaires de guerbet

(30) Priorität: 09.05.1995 DE 19516940
(43) Veröffentlichungstag der Anmeldung: 13.11.1996
(73) Patentinhaber: RWE-DEA AKTIENGESELLSCHAFT FÜR MINERALOEL UND CHEMIE, 22297 Hamburg (DE)
(72) Erfinder: Conrads, Martin, 47443 Moers (DE); Herrmann, Albert Thomas, 25541 Brunsbüttel (DE); Scherf, Erich, 25541 Brunsbüttel (DE); Wagner, Arwed, 47228 Duisburg (DE)
(74) Vertreter: Schupfner, Gerhard D.

(56) Entgegenhaltungen:
- EP-A- 0 211 552
- EP-A- 0 485 371
- DE-A- 1 950 604
- DE-A- 2 235 992
- DE-A- 2 625 945
- DE-B- 1 543 377
- US-A- 4 409 399

## Beschreibung

Die Erfindung betrifft ein Verfahren zur Herstellung von primären Aminen durch Umsetzung von in 2-Stellung verzweigten, primären Alkoholen, sogenannten Guerbetalkoholen, mit Ammoniak bei erhöhter Temperatur und unter Druck in der Flüssigphase in Gegenwart eines Metall-Katalysators/Katalysatorgemisches.

Primäre und sekundäre Fettamine spielen eine große Rolle als Rohstoffe für Tenside, Emulgatoren oder Mikrobiozide auf Basis von quartären Ammoniumsalzen. Durch Einführung verzweigter Alkylreste werden die physikalischen Eigenschatten der Endprodukte verbessert, da die Verzweigung in der Alkylkette zu einer Schmelzpunktabsenkung führt. Während dieser Effekt bereits durch Methylverzweigungen erreicht wird, führen längerkettige Verzweigungen in α-Stellung zur Aminfunktion zu zusätzlichen Eigenschaftsverbesserungen der Endprodukte. Bei Verwendung dieser Amine als Rohstoff für Wäschenachbehandlungsmittel ist ein günstiger Effekt auf die Wasseraufnahme zu verzeichnen, der sich in einer verbesserten Antistatikausrüstung und in einem höheren Tragekomfort der so behandelten Textilien äußert [DE-OS 2625945] gleichzeitig wird die bakterizide und fungizide Wirkung der Ammoniumsalze durch die Alkylverzweigung in α-Stellung zur Ammoniumfunktion drastisch erhöht [JP-OS 63096102], so daß man zu wirksamen Mikrobioziden gelangt, die sowohl als Desinfektionsmittel, als auch als Holz- und Bautenschutzmittel eingesetzt werden können. Ein weiteres Einsatzgebiet für Amine, die in α-Stellung verzweigt sind, stellen Aminoxide dar, die wegen ihrer Hautfreundlichkeit z. B. als Tenside in Körperpflegemitteln Verwendung finden.

Primäre Alkylamine mit langen Alkylresten, wie die hier beschriebenen Isofolamine, werden u. a. eingesetzt als Flotationshilfsmittel, Ölfeldchemikalien oder Korrosionsinhibitoren. Gleichzeitig stellen primäre Amine Synthesebausteine für eine Vielzahl von Derivaten dar, z.B. Fettaminethoxylate (Emulgatoren und Dispergiermittel), Betaine und Aminoxide (Reinigungsmittel) oder quaternäre Ammoniumverbindungen (Biozide, Antistatika) .

Die Umsetzung von Ammoniak mit primären, langkettigen Alkoholen ist bekannt. Zahlreiche für diese Flüssigphasenaminierung von Fettalkoholen mit Ammoniak geeignete Katalysatorsysteme sind beschrieben, beispielsweise in der US-PS 4409399, die den Stand der Technik zur Flüssigphasen-Aminierung beschreibt. Flüssigphasen-Aminierung bedeutet, daß der Alkohol sich in der flüssigen Phase befindet, während Ammoniak oder die primären bzw. sekundären Amine sich bei den angegebenen Reaktionsbedingungen entweder in der flüssigen oder in der Gasphase befinden. Es ist jedoch schwierig, selektiv ein spezifisches Amin, insbesondere ein primäres Amin, durch Umsetzung eines Alkohols mit Ammoniak herzustellen. Normalerweise erhält man große Mengen an sekundärem und tertiärem Amin, wenn man die Reaktion mit dem Ziel hoher Umsätze durchführt.

Zur Aminierung von Alkoholen wird eine Vielzahl unterschiedlicher metallischer Katalysatoren wie z.B. Ni, Cu, Cr, Co, und Fe bzw. deren Oxide, ggf. zusammen mit Alkali- und Erdalkalimetallen als Cokatalysatoren eingesetzt. Viele dieser Katalysatoren zeichnen sich durch eine hohe Reaktivität aus, die zu einer geringen Selektivität hinsichtlich der primären Amine führt.

In der DE-OS 2625945 wird die Aminierung von Guerbetalkoholen an einem Zinkchrom- oder Zinkaluminiumkontakt und Wasserstoff bei Temperaturen von 280°C bis 320°C und einem Druck von 250-260 bar beschrieben. Über die Selektivität hinsichtlich der primären Amine werden keine Aussagen gemacht. Die US-PS 4409399 verwendet dagegen Cu /Ni /Mg-Oxid -Katalysatorsysteme zur selektiven Herstellung sekundärer Fettamine.

Hohe Selektivitäten werden oftmals nur bei sehr geringen Umsätzen erzielt. Dies stellt aber immer dann einen entscheidenden Nachteil dar, wenn anschließend das primäre Amin durch Destillation vom Restalkohol getrennt werden muß. Speziell im Fall der Guerbetalkohole ist die destillative Trennung von Alkohol und primärem Amin wegen der geringen Siedepunktsdifferenz schwierig. Andererseits haben die üblichen Aminierungskatalysatoren bei höheren Umsatzraten den Nachteil einer geringen Selektivität hinsichtlich primärer Aminen.

Aufgabe der Erfindung ist es, ein Verfahren zur Verfügung zu stellen, das die selektive Herstellung von primären in 2-Stellung alkylverzweigten Aminen aus den entsprechenden Alkoholen mit einem geeigneten Katalysatorsystem unter geeigneten Reaktionsbedingungen erlaubt.

Überraschenderweise wurde festgestellt, daß sich Guerbetalkohole mit Ammoniak ungewöhnlich selektiv zu primären Guerbetaminen umsetzen lassen, wenn man als Katalysator/Cokatalysator-Systeme bestimmte Mischungen von Metallen bzw. deren Oxide mit Oxiden anderer Metalle als Cokatalysatoren einsetzt.

Das erfindungsgemäße Verfahren zur Herstellung von primären Aminen erfolgt durch Umsetzung von bestimmten Alkoholen mit Ammoniak in Gegenwart eines Katalysators / Katalysatorgemisches, indem man einen oder mehrere in 2-Stellung verzweigte, primäre Alkohol der Formel 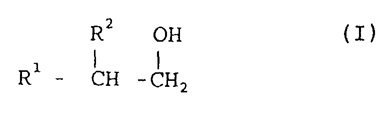 mit Ammoniak umsetzt, wobei die Reste R¹ und R² des (der) Alkohols(e) jeweils gleiche oder unterschiedliche aliphatische, gesättigte und unverzweigte, acyclische Alkylreste mit jeweils 3 bis 17 C-Atomen, sind wobei der Alkohol in der Summe aus 12 bis 36 C-Atomem besteht und der Katalysator / das Katalysatorgemisch geträgert ist und die Metalle Nickel und / oder Cobalt und / oder deren oxide enthält. Als weitere Komponente kann der Katalysator zusätzlich Kupfer und/oder Kupferoxid enthalten.

Die Katalysatoren und ggf. die Cokatalysatoren werden auf Kieselgel bzw. Silika, Tonerde- bzw. Aluminiumoxid und/oder Graphit bevorzugt aber auf SiO₂ als Träger fixiert.

Die Katalysatoren können zusammen mit Oxiden des Bariums Zirconiums und/oder Zinks als Cokatalysatoren eingesetzt werden.

Die Umsetzung der Alkohole mit Ammoniak kann in der Flüssigphase erfolgen, wobei die Umsetzung der Alkohole bei Temperaturen von 100 bis 350°C bevorzugt bei 230 bis 270°C durchgeführt wird. Das Mol-Verhältnis von Ammoniak zu Alkohol sollte 2,5:1 bis 5:1 betragen, wobei Verhältnisse von 3:1 bis 3,5:1 bevorzugt werden.

Zur Aktivitätssteigerung kann der Katalysators / das Katalysatorgemisch und ggf. ein oder mehrere Cokatalysator(en) mit Wasserstoff vor oder während der Umsetzung aktiviert werden. Die Umsetzung der Alkohole mit Ammoniak selbst wird bevorzugt in einer reduzierenden Atmosphäre von Wasserstoff bei einem Wasserstoffüberdruck von 1,05 bis 40 bar bevorzugt von 20 bis 40 bar durchgeführt. Bei einer Durchführungsform des erfindungsgemäßen Verfahren werden ein in α-Stellung verzweigter Alkohol mit 12 - 36 C-Atomen, und der Katalysator in einen Hochdruckautoklaven gefüllt. Nachdem die Luft im Reaktor durch Stickstoff verdrängt wurde, wird flüssiger Ammoniak eingefüllt. Bei Raumtemperatur wird mit Wasserstoff der Gesamtdruck auf 20 - 40 bar eingestellt. Anschließend wird auf die Reaktionstemperatur von 230 - 270°C erhitzt, wobei sich ein Druck von 100 bis 250 bar einstellt. Das Mol-Verhältnis von Ammoniak zu Alkohol sollte 2,5: 1 bis 5:1 betragen, wobei Verhältnisse von 3:1 bis 3,5:1 bevorzugt werden.

Nach der Reaktion wird der Autoklav abgekühlt und die Produkte werden nach Standardmethoden aufgearbeitet. Die Reaktion kann sowohl diskontinuierlich als auch kontinuierlich durchgeführt werden.

Als wirksam im Sinne der Erfindung haben sich Ni/NiO (Südchemie AG G49BRS) und Co/CoO (Südchemie AG G67BRS) Katalysatoren auf Silica erwiesen (siehe Tabelle 1) sowie ein Metall / Metalloxid-Katalysator aus Ni (42 Gew.%), Co(4 Gew.%) und Cu (4 Gew.%), der auf Kieselgur geträgert ist (Mallinckrodt E-221P).

**Tabelle 1:**

| Zusammensetzung der in den Beispielen 1 bis 3 eingesetzten Katalysatoren. | | | | |
|---|---|---|---|---|
| **Katalysatoren** | Zusammensetzung in Gew% ⁺ | | | |
| **G 67 RS** | **I** | Co / CoO (≈Σ60%) | ZrO₂ | SiO₂ |
| **G 49 B RS** | **II** | Ni(35%) / NiO(18%) | C(3%) | SiO₂(30%) |

| | | | | |
|---|---|---|---|---|
| ⁺ laut Merkblatt des Herstellers Südchemie AG | | | | |

Andere Katalysatoren/Cokatalysatoren haben sich für die selektive Umsetzung von Guerbetalkoholen zu primären Aminen nicht bewährt: Palladium/Aktivkohle (Aldrich), Kupfer-Chromit (Mallinckrodt E-106P), Cu/Zn (Mallinckrodt E-320P) und als trägerfreier Nickelkatalysator Mallinckrodt E-480D.

### Beispiele

Zur erfindungsgemäßen Durchführung der Reaktion wurde der Alkohol mit dem Ammoniak in Gegenwart des Katalysators unter den angegebenen Bedingungen zusammengebracht. Dabei kann die Überführung des Katalysators in die Wirkform entweder in situ oder in einer vorgelagerten Aktivierung in reduzierender Atmosphäre bei Temperaturen zwischen 150°C und 300°C erfolgen. Zur Gewinnung des gewünschten höheren Amins wurde am Ende der Umsetzung das Wasser und gegebenenfalls nicht umgesetztes Ammoniak durch Schnelldestillation aus dem Reaktionsgefäß ausgetrieben. Nach dem Abtrennen des heterogenen Katalysators von dem restlichen Reaktionsgemisch konnte man das gewünschte Amin abdestillieren. Im Destillationskolben verblieben als Rückstand die Kondensationsprodukte und schwerere Produkte zusammen mit nicht-umgesetztem Alkohol.

### Beispiele 1, 2 und 3

1 mol Guerbetalkohol und 3 g des Katalysators G 67 RS (I) der Südchemie wurden in einen Autoklaven gefüllt. Das System wurde mit Inertgas gespült, bevor 60 g verflüssigter Ammoniak eingefüllt wurde. Anschließend wurde mit Wasserstoff ein Druck von 20 bar eingestellt und der Reaktorinhalt unter Rühren auf 270°C erhitzt. Nach 4 Stunden bei dieser Temperatur wurde auf Raumtemperatur abgekühlt und entspannt. Der überschüssige Ammoniak wurde durch Erwärmen auf 50°C im Stickstoffstrom ausgetrieben, bevor die reinen Amine durch fraktionierte Destillation isoliert wurden. In Beispiel 3 wurde analog mit dem Katalysator G49BRS (II) umgesetzt.

**Tabelle 2 :**

| Aminierung verschiedener Guerbetalkohole | | | | | | |
|---|---|---|---|---|---|---|
| *Versuch* | | Zusammensetzung des Rohproduktes | | | | |
| | **Einsatz** | **Katalysator** | **Umsatz** | **prim. Amine**⁺ | **sek. Amine**⁺ | **andere**⁺***** |
| *1.* | **2-Hexyldecanol** | **I** | 93 % | **80,6%** | 6,8% | 12,6% |
| *2.* | **2-Octyldodecanol** | **I** | 91,7% | **83,5%** | 5,5% | 11 % |
| 3. | **2-Decyltetradecanol** | **II** | 97% | **88%** | - | 12% |

| | | | | | | |
|---|---|---|---|---|---|---|
| *) Restalkohol, tert. Amine, Amide, Nitrile | | | | | | |
| ⁺ Zusammensetzung in GC-Flächenprozenten | | | | | | |

## Patentansprüche

1. Verfahren zur Herstellung von primären Aminen durch Umsetzung von Alkoholen mit Ammoniak in Gegenwart eines Katalysators / Katalysatorgemisches, **dadurch gekennzeichnet,** daß
(A) man einen oder mehrere in 2-Stellung verzweigte, primäre Alkohole der Formel 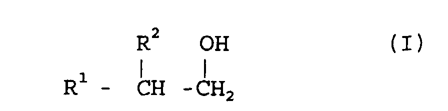 umsetzt, wobei R¹ und R², jeweils gleiche oder unterschiedliche aliphatische, gesättigte und unverzweigte, acyclische Alkylreste mit jeweils 3 bis 17 C-Atomen sind und der (die) Alkohol(e) in der Summe aus 12 bis 36 C-Atomem bestehen und
(B) der Katalysator/ das Katalysatorgemisch geträgert ist und die Metalle Nickel und/oder Cobalt und/oder deren Oxide enthält.

2. Verfahren gemäß Anspruch 1, **dadurch gekennzeichnet,** daß man den (die) Alkohol(e) in Gegenwart eines Katalysators / Katalysatorgemisches, das auf Siliciumdioxid, Aluminiumoxid und/oder Kohle, insbesondere auf Siliciundioxid, geträgert ist, umsetzt.

3. Verfahren gemäß einem der vorhergehenden Ansprüche, **dadurch gekennzeichnet,** daß man den (die) Alkohol(e) in Gegenwart eines Katalysators / Katalysatorgemisches, das Kupfer und / oder Kupferoxid enthält, umsetzt.

4. Verfahren gemäß einem der vorhergehenden Ansprüche, **dadurch gekennzeichnet,** daß man den (die) Alkohol(e) in Gegenwart eines oder mehrerer Cokatalysatoren, nämlich Oxiden des Zirconiums, Bariums und/oder Zinks umsetzt.

5. Verfahren gemäß einem der vorhergehenden Ansprüche, **dadurch gekennzeichnet,** daß man die Umsetzung mit Ammoniak in der Flüssigphase durchführt.

6. Verfahren gemäß einem der vorhergehenden Ansprüche, **dadurch gekennzeichnet,** daß man die Umsetzung bei Temperaturen von 100 bis 350°C, bevorzugt bei 230 bis 270°C durchführt.

7. Verfahren gemäß einem der vorhergehenden Ansprüche, **dadurch gekennzeichnet,** daß man die Umsetzung in Gegenwart eines Katalysators / Katalysatorgemisches und ggf. eines Cokatalysators durchführt, die vor oder während der Umsetzung mit Wasserstoff aktiviert wurden.

8. Verfahren gemäß einem der vorhergehenden Ansprüche, **dadurch gekennzeichnet,** daß man die Umsetzung in einer reduzierenden Atmosphäre durchführt, wobei zunächst Wasserstoff mit einem Wasserstoffüberdruck von 1,05 bis 40 bar, vorzugsweise 20 bis 40 bar bei Raumtemperatur eingestellt wird.

9. Verfahren gemäß einem der vorhergehenden Ansprüche, **dadurch gekennzeichnet,** daß man ein Mol-Verhältnis von Ammoniak zu Alkohol von 2,5: 1 bis 5:1, vorzugsweise von 3:1 bis 3,5:1, wählt.

## Claims

1. A process for producing primary amines by reacting alcohols with ammonia in the presence of a catalyst/ catalyst mixture characterized by
(A) reacting one or more primary alcohol(s) branched in position 2, as defined by the formula 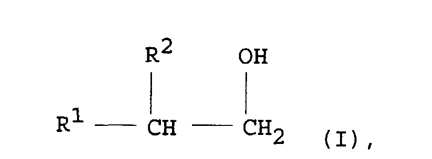 wherein R¹ and R² each represent the same or different aliphatic, saturated and unbranched, acyclic alkyl residues each having from 3 to 17 carbon atoms and the alcohol(s) in total consist(s) of 12 to 36 carbon atoms and
(B) the catalyst/catalyst mixture is supported and contains the metals nickel and/or cobalt and/or the oxides thereof.

2. The process of claim 1 characterized in that said alcohol(s) is (are) reacted in the presence of a catalyst/catalyst mixture supported on silica, alumina and/or charcoal, preferably on silica.

3. A process according to any one of the preceding claims characterized in that said alcohol(s) is (are) reacted in the presence of a catalyst/catalyst mixture containing copper and/or copper oxide.

4. A process according to any one of the preceding claims characterized in that said alcohol(s) is (are) reacted in the presence of one or more co-catalyst(s), namely zirconium oxide, barium oxide and/or zinc oxide.

5. A process according to any one of the preceding claims characterized in that the reaction is carried out with ammonia in the liquid phase.

6. A process according to any one of the preceding claims characterized in that the reaction is carried out at temperatures in the range of 100 to 350 °C, preferably 230 to 270 °C.

7. A process according to any one of the preceding claims characterized in that the reaction is carried out in the presence of a catalyst/catalyst mixture and, optionally, a co-catalyst and said catalyst(s) has (have) been activated with hydrogen prior to or during the reaction.

8. A process according to any one of the preceding claims characterized in that the reaction is carried out in a reducing atmosphere in which hydrogen at first is adjusted to an excess pressure of 1.05 to 40 bar, preferably 20 to 40 bar, at room temperature.

9. A process according to any one of the preceding claims characterized in that a molar relationship of ammonia to alcohol is selected of from 2,5:1 to 5:1, preferably of from 3:1 to 3,5:1.

## Revendications

1. Procédé de préparation d'amines primaires par transformation d'alcools avec de l'ammoniac en présence d'un catalyseur/mélange de catalyseurs, caractérisé en ce que
A) on transforme un ou plusieurs alcools primaires ramifiés en 2^{ème} position de formule 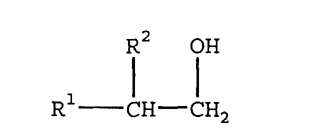 dans laquelle R¹ et R² sont chaque fois des radicaux alkyle identiques ou différents, aliphatiques, saturés et non ramifiés et acycliques avec chaque fois 3 à 17 atomes de carbone, et le ou les alcools constituent dans la somme de 12 à 36 atomes de carbone, et
B) le catalyseur/ mélange de catalyseurs est supporté et contient les métaux nickel et/ou cobalt et/ou leurs oxydes.

2. Procédé suivant la revendication 1, caractérisé en ce qu'on transforme le ou les alcools en présence d'un catalyseur/mélange de catalyseurs qui est supporté par du dioxyde de silicium, de l'oxyde d'aluminium et/ou du charbon, en particulier du dioxyde de silicium.

3. Procédé suivant l'une des revendications précédentes, caractérisé en ce qu'on transforme le ou les alcools en présence d'un catalyseur/mélange de catalyseurs, qui contient du cuivre et/ou de l'oxyde de cuivre.

4. Procédé suivant l'une des revendications précédentes, caractérisé en ce qu'on transforme le ou les alcools en présence d'un ou de plusieurs cocatalyseurs, à savoir des oxydes de zirconium, de baryum et/ou de zinc.

5. Procédé suivant l'une des revendications précédentes, caractérisé en ce qu'on effectue la transformation avec de l'ammoniac dans la phase liquide.

6. Procédé suivant l'une des revendications précédentes, caractérisé en ce qu'on effectue la transformation à des températures de 100 à 350°C, de préférence de 230 à 270°C.

7. Procédé suivant l'une des revendications précédentes, caractérisé en ce qu'on effectue la transformation en présence d'un catalyseur/mélange de catalyseur et, le cas échéant, d'un cocatalyseur, qui sont activés avec de l'hydrogène avant ou pendant la transformation.

8. Procédé suivant l'une des revendications précédentes, caractérisé en ce qu'on effectue la transformation dans une atmosphère réductrice, dans lequel on règle à température ambiante d'abord l'hydrogène avec une surpression d'hydrogène de 1,05 à 40 bars, de préférence de 20 à 40 bars.

9. Procédé suivant l'une des revendications précédentes, caractérisé en ce qu'on choisit un rapport molaire d'ammoniac à alcool de 2,5:1 à 5:1, de préférence de 3:1 à 3,5:1.
